# EUROPEAN PATENT APPLICATION

(11) **EP 4 364 814 A1**
(43) Date of publication of application: **08.05.2024**
(21) Application number: 22833247.4
(22) Date of filing: 29.06.2022
(51) Int. Cl.: A61Q 1/00, A61Q 19/00, A61K 8/11, A61K 8/25, A61K 8/44, B01J 13/14

(54) **AQUEOUS MICROCAPSULE DISPERSION**

(30) Priority: 30.06.2021 JP 2021109567
(71) Applicant: Kao Corporation, Chuo-ku Tokyo 103-8210 (JP)
(72) Inventor: KOGA, Yoshito, Wakayama-shi, Wakayama 640-8580 (JP); FUKUZUMI, Keita, Wakayama-shi, Wakayama 640-8580 (JP); KAMEYAMA, Yutaka, Wakayama-shi, Wakayama 640-8580 (JP); KONO, Hironori, Wakayama-shi, Wakayama 640-8580 (JP)
(74) Representative: Hoffmann Eitle
(86) International application number: PCT/JP2022/026086
(87) International publication number: WO 2023/277101

(57) **Abstract**

The present invention relates to an aqueous microcapsule dispersion liquid containing a component (A) and a component (B) below, a method of producing an aqueous microcapsule dispersion liquid, and a method of stabilizing an aqueous microcapsule dispersion liquid: component (A): microcapsules having a shell containing an inorganic material as a constitutional component, and a core containing one or more kind of an organic compound, inside the shell, and component (B): one or more kind selected from the group consisting of an amino acid (B1), a polyhydroxyamine (B2), and a quaternary ammonium hydroxide (B3).

## Description

### Field of Invention

The present invention relates to an aqueous microcapsule dispersion liquid, a method of producing an aqueous microcapsule dispersion liquid, and a method of stabilizing an aqueous microcapsule dispersion liquid.

### Background of the Invention

In a broad business field including cosmetics, medicines, general household products, and printing, various microcapsules encapsulating a fragrance material or a physiologically active substance therein have been developed and utilized. The methods having been used for producing the microcapsules include a chemical method, such as a suspension polymerization method, a mini-emulsion polymerization method, an emulsion polymerization method, a precipitation polymerization method, a dispersion polymerization method, an interfacial polycondensation method, and a submersible curing method; a physicochemical method, such as a submersible drying method, a phase-transfer emulsification method, and a coacervation method; and a mechanical method, such as a spray-drying method and a heteroaggregation method. Many of the production methods of microcapsules provide an aqueous dispersion liquid of microcapsules dispersed in an aqueous medium, and from the industrial standpoint, it is desired to use the resulting aqueous dispersion liquid of microcapsules directly without performing an isolation operation, such as filtration or drying.

Under the circumstances, various investigations have been made for aqueous dispersion liquids of microcapsules.

JP 2013-255915 A (PTL 1) intends to provide microcapsules including a core substance having a high concentration, and describes microcapsules including an active component as a core substance in which the microcapsules have a microcapsule shell constituted by an inorganic polymer formed by an in-situ polymerization method using a metal alkoxide or a semimetal alkoxide as a precursor utilizing an oil-in-water type emulsion method, and a suspension liquid formed of the microcapsules.

JP 2005-526025 A (PTL 2) describes a preparation method of an encapsulated lipophilic active substance composition in which an aqueous emulsion of the active substance composition is mixed with a water-reactive silicon compound to form a suspension liquid of microcapsules having a core of the active substance composition and a shell of a network polymer containing silicon as a major component, and the microcapsules are post-treated with a water-reactive metal alkoxy or acyloxy compound.

JP 2003-534249 A (PTL 3) describes a composition capable of stabilizing an active ingredient and capable of delivering the ingredient, containing microcapsules having a core-shell structure, the core containing the active ingredient, the core being encapsulated inside a microcapsule shell, the shell being formed of an inorganic polymer obtained by a sol-gel method.

### Summary of the Invention

The present invention relates to an aqueous microcapsule dispersion liquid containing a component (A) and a component (B) below.

Component (A): microcapsules having a shell containing an inorganic material as a constitutional component, and a core containing one or more kind of an organic compound, inside the shell

Component (B): one or more kind selected from the group consisting of an amino acid (B1), a polyhydroxyamine (B2), and a quaternary ammonium hydroxide (B3)

### Detailed Description of the Invention

It has been found that the techniques of PTLs 1 to 3 cannot retain the encapsulated organic compound, such as a fragrance material, for a long period of time in some cases. Furthermore, the microcapsules may be finally released to the natural environment. Therefore, there is a demand of an aqueous dispersion liquid of microcapsules that can reduce the burden on the natural environment, and is excellent in retainability of an encapsulated organic compound, such as a fragrance material, even in long-term storage.

The present invention relates to an aqueous microcapsule dispersion liquid that is capable of reducing the burden on the natural environment, and capable of retaining an encapsulated organic compound, such as a fragrance material, as an active ingredient for a long period of time, a method of producing an aqueous microcapsule dispersion liquid, and a method of stabilizing an aqueous microcapsule dispersion liquid.

The present inventors have focused the fact that in an aqueous dispersion liquid of microcapsules having a shell containing an inorganic material as a constitutional component and a core containing one or more kind of an organic compound, inside the shell, the organic compound can be retained for a long period of time by containing one or more kind selected from the group consisting of an amino acid, a polyhydroxyamine, and a quaternary ammonium hydroxide, and thus have found that an aqueous microcapsule dispersion liquid that is capable of reducing the burden on the natural environment, and capable of retaining an encapsulated organic compound, such as a fragrance material, as an active ingredient for a long period of time, a method of producing an aqueous microcapsule dispersion liquid, and a method of stabilizing an aqueous microcapsule dispersion liquid can be provided.

Specifically, the present invention relates to the following items [1] to [3].
[1] An aqueous microcapsule dispersion liquid containing a component (A) and a component (B) below:
   component (A): microcapsules having a shell containing an inorganic material as a constitutional component, and a core containing one or more kind of an organic compound, inside the shell, and
   component (B): one or more kind selected from the group consisting of an amino acid (B1), a polyhydroxyamine (B2), and a quaternary ammonium hydroxide (B3).
[2] A method of producing an aqueous microcapsule dispersion liquid containing a component (A) and a component (B) below:
   component (A): microcapsules having a shell containing an inorganic material as a constitutional component, and a core containing one or more kind of an organic compound, inside the shell, and
   component (B): one or more kind selected from the group consisting of an amino acid (B1), a polyhydroxyamine (B2), and a quaternary ammonium hydroxide (B3),
   the method of producing an aqueous microcapsule dispersion liquid including a step of adding the component (B) to an aqueous microcapsule dispersion containing the component (A).
[3] A method of stabilizing an aqueous microcapsule dispersion liquid containing a component (A) and a component (B) below:
   component (A): microcapsules having a shell containing an inorganic material as a constitutional component, and a core containing one or more kind of an organic compound, inside the shell, and
   component (B): one or more kind selected from the group consisting of an amino acid (B1), a polyhydroxyamine (B2), and a quaternary ammonium hydroxide (B3),
   the method of stabilizing an aqueous microcapsule dispersion liquid including adding the component (B) to an aqueous microcapsule dispersion containing the component (A), so as to stabilize the aqueous microcapsule dispersion liquid.

The present invention can provide an aqueous microcapsule dispersion liquid that is capable of reducing the burden on the natural environment, and capable of retaining an encapsulated organic compound, such as a fragrance material, as an active ingredient for a long period of time, a method of producing an aqueous microcapsule dispersion liquid, and method of stabilizing an aqueous microcapsule dispersion liquid.

### [Aqueous Microcapsule Dispersion Liquid]

The aqueous microcapsule dispersion liquid (which may be hereinafter referred simply to as an "aqueous dispersion liquid") of the present invention containing a component (A) and a component (B) below.
Component (A): microcapsules having a shell containing an inorganic material as a constitutional component, and a core containing one or more kind of an organic compound, inside the shell
Component (B): one or more kind selected from the group consisting of an amino acid (B1), a polyhydroxyamine (B2), and a quaternary ammonium hydroxide (B3)

The aqueous microcapsule dispersion liquid of the present invention contains the microcapsules as the component (A) dispersed in an aqueous medium.

In the description herein, the "aqueous medium" is a liquid containing at least water, and preferably contains water occupying the largest proportion in the medium. Examples of the component other than water capable of being contained in the aqueous medium include an aliphatic alcohol having 1 or more and 4 or less carbon atoms; a ketone compound having 3 or more and 8 or less carbon atoms; an ether compound, such as ethyl ether and tetrahydrofuran; and an ester compound, such as methyl acetate.

The content of water in the aqueous medium is preferably 80% by mass or more, and more preferably 90% by mass or more, is 100% by mass or less, and further preferably 100% by mass, from the standpoint of enhancing the dispersion stability and the particle diameter stability of the microcapsules. Preferred examples of water used include ion exchanged water, deionized water, and distilled water.

In the description herein, the retainability of the encapsulated organic compound for a long period of time may be referred to as "long-term retainability".

In the present invention, the expression "containing the component (A) and the component (B)" also means "formed by blending the component (A) and the component (B)".

The reason why the effects of the present invention can be attained, while not entirely clear, will be described with reference to the case of silica microcapsules having a shell containing silica as a constitutional component under an alkaline environment as an example.

In general, in the case where the aqueous microcapsule dispersion liquid is under an alkaline environment, it is estimated that the long-term retainability of the encapsulated organic compound is deteriorated.

However, it is considered that the fact that the aqueous microcapsule dispersion liquid is under an alkaline environment advantageously contributes to the condensation reaction of silica. In the present invention, it is considered that the nitrogen atom of an amino acid, a polyhydroxyamine, or a quaternary ammonium hydroxide contained in the aqueous dispersion liquid is positively charged, the oxygen atom of the amino acid, the polyhydroxyamine, or the quaternary ammonium hydroxide is negatively charged, the functional group or the ion of the amino acid, the polyhydroxyamine, or the quaternary ammonium hydroxide is located at the surface of the silica microcapsules, and the positively charged nitrogen atom and the negatively charged oxygen atom may interact with each other to contribute to the condensation reaction of silica, resulting in a dense and firm shell, which suppresses the encapsulated organic compound from being leaked, thereby enhancing the long-term retainability.

In the present invention, the shell of the microcapsules contains an inorganic material as a constitutional component, and thereby the burden on the natural environment can also be reduced.

### <Component (A)>

The aqueous microcapsule dispersion liquid of the present invention contains microcapsules having a shell containing an inorganic material as a constitutional component, and a core containing one or more kind of an organic compound, inside the shell, as the component (A), from the standpoint of reducing the burden on the natural environment in releasing to the external environment.

The inorganic material constituting the shell of the component (A) is preferably a metal oxide containing a metal element or a semimetal element, and more preferably an inorganic polymer formed through sol-gel reaction using a metal alkoxide (M(OR)ₓ) as a shell precursor, in which M represents a metal or a semimetal, and R represents a hydrocarbon group. Examples of the metal or semimetal element constituting the metal alkoxide include silicon, aluminum, titanium, zirconium, and zinc.

The inorganic material is further preferably an inorganic polymer formed through sol-gel reaction using a metal alkoxide of one or more kind selected from the group consisting of silicon, aluminum, and titanium as a shell precursor, and still further preferably silica formed through sol-gel reaction using an alkoxysilane as a shell precursor, from the standpoint of enhancing the long-term retainability, and the standpoint of reducing the burden on the natural environment in releasing to the external environment. Accordingly, the component (A) is preferably microcapsules having a shell containing silica as a constitutional component, and a core containing one or more kind of an organic compound, inside the shell (i.e., silica microcapsules) (which may be hereinafter referred to as "silica capsules").

The alkoxysilane is preferably a tetraalkoxysilane from the standpoint of enhancing the long-term retainability.

The tetraalkoxysilane is preferably a tetraalkoxysilane having an alkoxy group having 1 or more and 4 or less carbon atoms, more preferably one or more kind selected from the group consisting of tetramethoxysilane, tetraethoxysilane, and tetraisopropoxysilane, further preferably one or more kind selected from the group consisting of tetramethoxysilane and tetraethoxysilane, and still further preferably tetraethoxysilane, from the same standpoint as above.

The organic compound contained in the core of the component (A) is preferably one or more kind selected from the group consisting of a fragrance material; a fragrance precursor; an oil; an antioxidant; an antibacterial agent; a fertilizer; a surface modifier for fibers, skin, hair, and the like; a cooling agent; a dye; a colorant; a silicone; a solvent; and an oil soluble polymer, more preferably one or more kind selected from the group consisting of a fragrance material, a fragrance precursor, an oil, an antioxidant, an antibacterial agent, a fertilizer, a surface modifier, and a solvent, further preferably one or more kind selected from the group consisting of a fragrance material, a fragrance precursor, an oil, an antioxidant, and a solvent, still further preferably one or more kind selected from the group consisting of a fragrance material, a fragrance precursor, and an oil, and still more further preferably one or more kind selected from the group consisting of a fragrance material and a fragrance precursor.

The organic compounds may be appropriately combined depending on the application of the microcapsules.

Examples of the fragrance precursor include a compound releasing a fragrance component through reaction with water, and a compound releasing a fragrance component through reaction with light.

Examples of the compound releasing a fragrance component through reaction with water include a silicate ester compound having an alkoxy component derived from a fragrance alcohol, a fatty acid ester compound having an alkoxy component derived from a fragrance alcohol, an acetal compound or a hemiacetal compound obtained through reaction of a carbonyl component derived from a fragrance aldehyde or a fragrance ketone and an alcohol compound, a Schiff base compound obtained through reaction of a carbonyl component derived from a fragrance aldehyde or a fragrance ketone and a primary amine compound, and a hemiaminal compound or a hydrazone compound obtained through reaction of a carbonyl component derived from a fragrance aldehyde or a fragrance ketone and a hydrazine compound.

Examples of the compound releasing a fragrance component through reaction with light include a 2-nitrobenzyl ether compound having an alkoxy component derived from a fragrance alcohol, an α-keto ester compound having a carbonyl component derived from a fragrance aldehyde or a fragrance ketone, and a coumarate ester compound having an alkoxy component derived from a fragrance alcohol. These fragrance precursors each may be, for example, a polymer, such as a reaction product of a part of a carboxy group of a polyacrylic acid and a fragrance alcohol.

The organic compound preferably has appropriate hydrophobicity from the standpoint of the long-term retainability. As an index showing the hydrophilicity or hydrophobicity of the organic compound, a cLogP value that is the calculated value of common logarithm of the distribution coefficient P between n-octanol and water (n-octanol/water) "logP" may be used. The cLogP value herein is the "LogP (cLogP)" calculated by the method described in A. Leo Comprehensive Medicinal Chemistry, Vol. 4 C. Hansch, P.G. Sammens, J.B. Taylor and C.A. Ramsden, Eds., P. 295, Pergamon Press, 1990, and is a cLogP value that is calculated with a program, CLOGP v. 4.01.

In the case where the organic compound is constituted by multiple kinds of constitutional components, the cLogP value of the organic compound can be calculated in such a manner that the cLogP values of the constitutional components are multiplied by the volume proportions of the constitutional components respectively, and the resulting values are summated. In this calculation method, all the constitutional components that each have a content of 0.5% by mass or more in the organic compound are considered. Even the constitutional component that has a content of less than 0.5% by mass in the organic compound may be included in the calculation in the case where the specific gravity and the cLogP value thereof are known.

The cLogP value of the organic compound is preferably 1 or more, more preferably 2 or more, and further preferably 3 or more, and is also preferably 30 or less, more preferably 20 or less, and further preferably 10 or less.

The shell of the silica capsules encapsulates the core, contains silica as a constitutional component, and preferably has an average thickness of 5 nm or more and 20 nm or less.

The shell of the silica capsules is preferably a multilayer shell including an inner shell containing silica, which is a hydrolytic polycondensation product of an alkoxysilane, as a constitutional component, and an outer shell containing silica, which is a hydrolytic polycondensation product of an alkoxysilane, as a constitutional component, outside the inner shell, from the standpoint of enhancing the dispersion stability of the microcapsules, and enhancing the long-term retainability. Specific examples of the silica capsules include the silica capsules described in JP 2015-128762 A.

In the case where the shell of the silica capsules is the multilayer shell including the inner shell and the outer shell, the inner shell encapsulates the core, contains silica as a constitutional component, and preferably has an average thickness of 5 nm or more and 20 nm or less, and the outer shell encapsulates the inner shell, contains silica as a constitutional component, and preferably has an average thickness of 10 nm or more and 100 nm or less.

The average thickness of the shell of the silica capsules and the average thicknesses of the inner shell and the outer shell of the silica capsules can be measured with a transmission electron microscope (TEM). Specifically, under observation with a transmission electron microscope, the thicknesses of the shell or the inner shell and the outer shell are actually measured on the micrograph. This operation is repeated five times while changing the view fields. The distribution of the average thickness of the shell or the inner shell and the outer shell is obtained from the resulting data. The magnification of the transmission electron microscope is 10,000 or more and 100,000 or less as a rough standard, and may be appropriately regulated depending on the size of the silica capsules. Examples of the transmission electron microscope (TEM) used herein include "JEM-2100", trade name, available from JEOL, Ltd.

The component (A) used may be appropriately synthesized.

In the case where the component (A) is silica capsules, preferred examples of the silica capsules include those produced by a method including the following step I.

Step I: A step of subjecting an emulsion liquid obtained by emulsifying a water phase component containing a cationic surfactant and an oil phase component containing the organic compound and a tetraalkoxysilane, to sol-gel reaction under an acidic condition, so as to form silica capsules having a core and a shell containing silica as a constitutional component, resulting in an aqueous dispersion containing the silica capsules

Examples of the cationic surfactant used in the step I include an alkylamine salt and an alkyl quaternary ammonium salt. The number of carbon atoms of the alkylamine salt and the alkyl quaternary ammonium salt is preferably 10 or more and 22 or less.

The content of the cationic surfactant in the water phase component in the step I is preferably 0.05% by mass or more and 10% by mass or less, from the standpoint of the dispersion stability of the emulsion droplets.

The amount of the tetraalkoxysilane used in the step I is preferably 10 parts by mass or more from the standpoint of accelerating the sol-gel reaction and forming a sufficiently dense shell, and is also preferably 60 parts by mass or less from the standpoint of suppressing the tetraalkoxysilane from remaining in the organic compound, all per 100 parts by mass of the organic compound used in the step I.

The amount of the oil phase component in the total amount of the emulsion liquid used in the step I is preferably 5% by mass or more and 50% by mass or less from the standpoint of the production efficiency.

The agitation means used in the preparation of the emulsion liquid is not particularly limited, and a homogenizer, a high pressure disperser, an ultrasonic disperser, and the like, having a large shearing force may be used.

The temperature in mixing and emulsifying the water phase component and the oil phase component is preferably 5°C or more and 50°C or less from the standpoint of the production stability.

The rotation number and the like of the agitation means and the period of time of mixing and emulsifying the water phase component and the oil phase component may be appropriately regulated to provide a median diameter D₅₀ of emulsion droplets of the emulsion liquid within the range described later.

The median diameter D₅₀ of the emulsion droplets of the emulsion liquid in the step I is preferably 0.1 µm or more from the standpoint of reducing the specific surface area of the silica capsules facing the external environment, and enhancing the retention of the organic compound, and is also preferably 50 µm or less from the standpoint of reducing the particle diameter of the silica capsules and the standpoint of the mechanical strength of the silica capsules.

The median diameter D₅₀ of the emulsion droplets can be measured according to the method shown in the examples.

The initial pH of the sol-gel reaction in the step I is preferably 3.0 or more from the standpoint of retaining the balance between the hydrolytic decomposition reaction and the condensation reaction of the tetraalkoxysilane and the standpoint of suppressing the sol having high hydrophilicity and accelerating the progress of the capsulation reaction, and is also preferably 4.5 or less from the standpoint of suppressing the simultaneous occurrence of the formation of the silica shell and the aggregation of the emulsion droplets, and providing the silica capsules having a dense shell.

The pH of the emulsion liquid may be regulated by using a pH modifier depending on the strength of acidity or alkalinity of the oil phase component containing the organic compound from the standpoint of regulating to the desired initial pH.

Examples of the acidic pH modifier include an inorganic acid, such as hydrochloric acid, nitric acid, and sulfuric acid, an organic acid, such as acetic acid and citric acid, and a liquid obtained by adding a cation exchange resin or the like to water, ethanol, or the like, and one or more kind selected from the group consisting of hydrochloric acid, sulfuric acid, nitric acid, and citric acid is preferred.

Examples of the alkaline pH modifier include sodium hydroxide, sodium hydrogen carbonate, potassium hydroxide, ammonium hydroxide, monoethanolamine, diethanolamine, triethanolamine, and trishydroxymethylaminomethane, and one or more kind selected from the group consisting of sodium hydroxide and ammonium hydroxide is preferred.

The pH of the emulsion liquid may have pH that is a desired value or less, and in this case, it is preferred to regulate by using the alkaline pH modifier.

The reaction temperature of the sol-gel reaction in the step I may be an optional value selected from the melting point of water contained in the water phase or more and the boiling point thereof or less, and the temperature is preferably in a certain range from the standpoint of regulating the balance between the hydrolytic decomposition reaction and the condensation reaction in the sol-gel reaction and forming the dense shell. The range is preferably 5°C or more and 60°C or less, and more preferably 10°C or more and 50°C or less.

The reaction time of the sol-gel reaction in the step I is preferably 0.5 hour or more and 50 hours or less assuming that the time when the temperature in the reaction system becomes the predetermined reaction temperature is the start of reaction.

In the case where the shell of the silica capsules is the aforementioned multilayer shell, one specific example of the silica capsules preferably contains, as a constitutional component, silica that is formed in such a manner that a tetraalkoxysilane as a silica precursor is further added to the aqueous dispersion containing the silica capsules obtained through the step I (which are hereinafter referred to as silica capsules (1)), so as to perform the sol-gel reaction in two steps. Accordingly, the silica capsules in this case are preferably produced by a method including the step 1 and the step 2 shown below.

Step 1: A step of subjecting an emulsion liquid obtained by emulsifying a water phase component containing a cationic surfactant and an oil phase component containing the organic compound and a tetraalkoxysilane, to sol-gel reaction under an acidic condition, so as to form silica capsules (1) having a core and a first shell containing silica as a constitutional component, resulting in an aqueous dispersion containing the silica capsules (1)

Step 2: A step of further adding a tetraalkoxysilane to the aqueous dispersion containing the silica capsules (1) obtained in the step 1, so as to perform sol-gel reaction, resulting in silica capsules having a second shell encapsulating the first shell

In the description herein, the expression "encapsulating the first shell" in the case where the step 1 and the step 2 are performed means encapsulating the first shell of the silica capsules (1) formed in the step 1, and also includes encapsulating the silica capsules (1).

The step 2 further forms a shell on the silica capsules formed in the step 1, and the silica capsules obtained in the step 2 become silica capsules having a shell with an increased thickness in the entirety thereof, which are considered to be silica capsules having a shell including the shell formed in the step 1 as an inner shell and the shell formed in the step 2 as an outer shell.

The step 1 may be performed in the same procedure as in the step I described above.

The amount of the tetraalkoxysilane used in the step 2 is preferably 7 parts by mass or more from the standpoint of forming the second shell encapsulating the first shell, and is also preferably 200 parts by mass or less from the standpoint of suppressing the formation of silica sol dispersed in the water phase, enhancing the dispersion stability of the silica capsules, and enhancing the long-term retainability, all per 100 parts by mass of the organic compound used in the step 1.

The total amount of the tetraalkoxysilane used in the case where the step 1 and the step 2 are performed, i.e., the total amount of the tetraalkoxysilane used in the step 1 and the step 2, is preferably 30 parts by mass or more and is also preferably 250 parts by mass or less per 100 parts by mass of the organic compound used in the step 1.

The median diameter D₅₀ of the microcapsules as the component (A) is preferably 100 µm or less, more preferably 75 µm or less, further preferably 50 µm or less, still further preferably 30 µm or less, and still more further preferably 10 µm or less, from the standpoint of enhancing the dispersion stability according to the Stokes' law and enhancing the long-term retainability, and is also preferably 0.01 µm or more, more preferably 0.05 µm or more, further preferably 0.07 µm or more, still further preferably 0.1 µm or more, still more further preferably 0.5 µm or more, and even further preferably 1 µm or more, from the standpoint of reducing the specific surface area of the microcapsules, and enhancing the long-term retainability. The median diameter D₅₀ of the component (A) can be measured according to the method shown in the examples.

The difference in specific gravity between the component (A) and the aqueous medium is preferably less than 0.30, more preferably less than 0.20, further preferably less than 0.15, still further preferably less than 0.10, still more further preferably less than 0.05, and even further preferably less than 0.01, and it is even still further preferred that there is no difference in specific gravity between them, from the standpoint of suppressing the upwelling or sedimentation of the microcapsules with the lapse of time, enhancing the dispersion stability, and enhancing the long-term retainability.

The specific gravity of the component (A) is determined by the specific gravities of the shell and the core constituting the component (A), and the mass ratio of the shell and the core.

### <Component (B)>

The aqueous microcapsule dispersion liquid of the present invention contains one or more kind selected from the group consisting of an amino acid (B1), a polyhydroxyamine (B2), and a quaternary ammonium hydroxide (B3) as the component (B) from the standpoint of enhancing the long-term retainability.

The component (B) may have a buffering effect capable of retaining the pH of the aqueous microcapsule dispersion liquid of the present invention to an alkaline environment from the standpoint of enhancing the long-term retainability. The component (B) preferably can retain the pH at 25°C of the aqueous microcapsule dispersion liquid of the present invention to 7.5 or more and 11.0 or less from the same standpoint as above.

One kind of the component (B) may be used alone, or two or more kinds thereof may be used in combination.

The acid dissociation exponent pKa at 25°C of at least one dissociation stages of the component (B) is preferably 7.5 or more, more preferably 8.0 or more, and further preferably 9.0 or more, and is also preferably 13.0 or less, more preferably 12.0 or less, further preferably 11.0 or less, and still further preferably 10.0 or less, from the standpoint of retaining the pH of the aqueous microcapsule dispersion liquid to an alkaline environment, and enhancing the long-term retainability.

In the present invention, it suffices that acid dissociation exponent pKa of at least one dissociation stages of the component (B) is in the aforementioned range. Specifically, in the case where component (B) has multiple dissociation stages, it suffices that the acid dissociation exponent pKa of at least one of the dissociation stages is in the aforementioned range.

The acid dissociation exponent pKa at 25°C of the component (B) used in the present invention is a pKa value of a conjugated acid calculated by "Chemicalize", an online platform for chemical calculations, search, and text processing (https://chemicalize.com/welcome), available from ChemAxon Ltd. Specifically, the calculation of pKa using Chemicalize can be performed by entering the chemical structure of the target compound by the SMILES notation in the form of single line character string.

Examples of the amino acid (B1) include a neutral amino acid, an acidic amino acid, and a basic amino acid.

Examples of the neutral amino acid include an aliphatic amino acid, an aromatic amino acid, and a heterocyclic amino acid. Among these, an aliphatic amino acid is preferred.

Examples of the aliphatic amino acid include a monoaminomonocarboxylic acid, such as glycine, alanine, valine, leucine, and isoleucine; a hydroxymonoaminomonocarboxylic acid, such as serine and threonine; a monoaminodicarboxylic acid, such as asparaginic acid and glutamic acid; a diaminomonocarboxylic acid, such as an acid amide amino acid, e.g., asparagine and glutamine; and a sulfur-containing amino acid, such as cysteine, cystine, and methionine.

Examples of the acidic amino acid include glutamic acid and asparaginic acid.

Examples of the basic amino acid include lycine, hydroxylycine, histidine, and arginine.

Preferred examples of the polyhydroxyamine (B2) include an organic compound having one amino group and two or more hydroxy groups.

Examples of the amino group include a primary amino group (-NH₂), a secondary amino group (-NHR¹, =NH (imino group)), and a tertiary amino group (-NR¹R²), wherein R¹ to R³ each represent a methyl group, an ethyl group, a propyl group, or an isopropyl group.

Preferred specific examples of the polyhydroxyamine (B2) include one or more kind selected from the group consisting of dimethanolamine, trimethanolamine, diethanolamine, triethanolamine, N-methyldiethanolamine, dipropanolamine, tripropanolamine, diisopropanolamine, triisopropanolamine, trishydroxymethylaminomethane, and 2-amino-2-methyl-1,3-propanediol. Among these, the polyhydroxyamine (B2) is more preferably one or more kind selected from the group consisting of diethanolamine, triethanolamine, and trishydroxymethylaminomethane, and further preferably trishydroxymethylaminomethane.

The quaternary ammonium hydroxide (B3) is preferably a tetraalkylammonium hydroxide. Preferred specific examples of the quaternary ammonium hydroxide (B3) include one or more kind selected from the group consisting of tetramethylammonium hydroxide (TMAH), tetraethylammonium hydroxide (TEAH), tetrabutylammonium hydroxide (TBAH), tetrahexylammonium hydroxide (THAH), and benzyltrimethylammonium hydroxide (BTMAH). Among these, the quaternary ammonium hydroxide (B3) is more preferably tetramethylammonium hydroxide.

Among the above, the component (B) is preferably one or more kind selected from the group consisting of the amino acid (B1) and the polyhydroxyamine (B2), more preferably contains the amino acid (B1), and further preferably is the amino acid (B1), from the standpoint of retaining the pH of the aqueous microcapsule dispersion liquid to an alkaline environment, and enhancing the long-term retainability.

In the case where the component (B) contains the amino acid (B1), the amino acid (B1) is preferably one or more kind selected from the group consisting of a neutral amino acid and an acidic amino acid, more preferably a neutral amino acid, further preferably a monoaminomonocarboxylic acid, still further preferably one or more kind selected from the group consisting of glycine and alanine, and still more further preferably glycine.

### <Component (C)>

The aqueous microcapsule dispersion liquid of the present invention preferably further contains an alkaline agent (provided that the component (B) is excluded) as a component (C) from the standpoint of enhancing the long-term retainability.

In the present invention, the expression "further containing the component (C)" also shows "formed by further blending the component (C)".

The component (C) is preferably contained or blended for regulating the aqueous microcapsule dispersion liquid to be under a desired alkaline environment.

The component (C) may be an inorganic alkaline agent or an organic alkaline agent, and is not particularly limited, as far as excluding the component (B). One kind of the component (C) may be used alone, or two or more kinds thereof may be used in combination.

Examples of the inorganic alkaline agent include a hydroxide of an alkali metal, such as sodium hydroxide and potassium hydroxide; a silicate of an alkali metal, such as potassium silicate (e.g., No. 1 potassium silicate, No. 2 potassium silicate, potassium orthosilicate, and potassium metasilicate) and sodium silicate (e.g., No. 1 sodium silicate, No. 2 sodium silicate, sodium orthosilicate, and sodium metasilicate); a carbonate of an alkali metal, such as disodium carbonate, sodium hydrogen carbonate, and dipotassium carbonate; a phosphate of an alkali metal, such as trisodium phosphate; a borate of an alkali metal, such as sodium borate; and ammonia.

Examples of the organic alkali agent include an organic amine, such as a monohydroxyalkylamine and an alkylamine.

Examples of the monohydroxyalkylamine include monomethanolamine, monoethanolamine, N-methylethanolamine, monopropanolamine, monoisopropanolamine, N-methylpropanolamine, N-(2-aminoethyl)ethanolamine, and 2-amino-2-methyl-1-propanol.

Examples of the alkylamine include monomethylamine, dimethylamine, monoethylamine, diethylamine, triethylamine, mono-n-propylamine, monoisopropylamine, diisopropylamine, triisopropylamine, mono-n-butylamine, mono-tert-butylamine, mono-sec-butylamine, mono-2-ethylhexylamine, tri-n-octylamine, and N-methylethylamine.

The total number of carbon atoms of the organic amine is preferably 1 or more, and more preferably 2 or more, and is also preferably 10 or less, more preferably 8 or less, further preferably 6 or less, and still further preferably 4 or less.

Among these, the component (C) is preferably one or more kind selected from the group consisting of a hydroxide, a silicate, a carbonate, and a phosphate of an alkali metal, and an organic amine, more preferably one or more kind selected from the group consisting of a silicate of an alkali metal and a monohydroxyalkylamine having 1 or more and 10 or less carbon atoms in total, further preferably one or more kind selected from the group consisting of sodium silicate and a monohydroxyalkylamine having 2 or more and 6 or less carbon atoms in total, and still further preferably one or more kind selected from the group consisting of No. 2 sodium silicate and monoethanolamine, from the standpoint of the easiness of regulation to a desired alkaline environment, the availability, the economic efficiency, and the like.

### <Component (D)>

The aqueous microcapsule dispersion liquid of the present invention preferably further contains a dispersant as a component (D) from the standpoint of enhancing the dispersion stability of the microcapsules.

In the present invention, the expression "further containing the component (D)" also shows "formed by further blending the component (D)".

Examples of the component (D) include an anionic surfactant and a nonionic surfactant.

One kind of the component (D) may be used alone, or two or more kinds thereof may be used in combination.

The anionic surfactant has an anionic group as a hydrophilic and a lipophilic group in the molecule from the standpoint of enhancing the long-term retainability. Examples of the anionic group include a group releasing a hydrogen ion through dissociation, such as a sulfuric acid ester group (-OSO₃M), a sulfonic acid group (-SO₃M), a carboxy group (-COOM), a phosphoric acid group (-OPO₃M₂), and ionic forms thereof formed through dissociation (such as -OSO₃⁻, -SO₃⁻, -COO⁻, -OPO₃²⁻, and -OPO₃⁻M). In the chemical formulae, M represents a counter ion of the anionic group.

Examples of the counter ion of the anionic group of the anionic surfactant include an alkali metal ion, such as a sodium ion and a potassium ion; an alkaline earth metal ion, such as a calcium ion and a magnesium ion; an ammonium ion; and an alkanolammonium having 1 to 3 alkanol groups having 2 or 3 carbon atoms (such as monoethanolammonium, diethanolammonium, triethanolammonium, and triisopropanolammonium).

Examples of the anionic surfactant include a low molecular weight dispersant having an anionic group and a high molecular weight dispersant having an anionic group.

Examples of the low molecular weight dispersant having an anionic group include a sulfonate, such as a linear alkylbenzenesulfonate (LAS); a sulfuric acid ester salt, such as an alkylsulfuric acid ester salt, a polyoxyethylene alkylethersulfuric acid ester salt, and a polyoxyethylene polyoxypropylene alkylethersulfuric acid ester salt; and a carboxylic acid salt, such as an alkenyl succinic acid salt and a fatty acid salt having 8 or more and 22 or less carbon atoms.

Among these, the low molecular weight dispersant having an anionic group is preferably a linear alkylbenzenesulfonate. Specific examples of the linear alkylbenzenesulfonate include sodium dodecylbenzenesulfonate.

The high molecular weight dispersant having an anionic group is preferably a high molecular weight dispersant having one or more kind of an anionic group selected from the group consisting of a carboxy group and a sulfonic acid group.

The high molecular weight dispersant having a carboxy group preferably contains a structural unit derived from one or more kind of a carboxy group-containing vinyl monomer selected from the group consisting of acrylic acid, methacrylic acid, maleic aid, and maleic anhydride, and more preferably one or more kind selected from the group consisting of an acrylic acid homopolymer, a methacrylic acid homopolymer, an acrylic acid-maleic acid copolymer, a methacrylic acid-maleic acid copolymer, an acrylic acid-maleic anhydride copolymer, a methacrylic acid-maleic anhydride copolymer, and salts thereof.

Examples of the high molecular weight dispersant having a sulfinic acid group include a salt of an aromatic sulfonic acid formalin condensate, such as a β-naphthalenesulfonic acid formalin condensate.

Among these, the high molecular weight dispersant having an anionic group is more preferably one or more kind selected from a high molecular weight dispersant having a carboxy group, and more preferably one or more kind selected from the group consisting of an acrylic acid-maleic acid copolymer, an acrylic acid-maleic anhydride copolymer, and salts thereof.

Examples of the nonionic dispersant include a nonionic surfactant.

Examples of the nonionic surfactant include a polyoxyethylene alkyl ether having an alkyl group having 8 or more and 22 or less carbon atoms, a sorbitol fatty acid ester, a sorbitan fatty acid ester, a glycerin fatty acid ester, a pentaerythritol fatty acid ester, a polyoxyethylene fatty acid ester, an alkylphenol ethylene oxide adduct, a higher alkylamine ethylene oxide adduct, and a polypropylene glycol ethylene oxide adduct.

As described above, the component (D) is preferably an anionic surfactant, more preferably one or more kind selected from the group consisting of a low molecular weight dispersant having an anionic group and a high molecular weight dispersant having an anionic group, further preferably one or more kind selected from the group consisting of a linear alkylbenzenesulfonate and a high molecular weight dispersant having one or more kind of an anionic group selected from the group consisting of a carboxy group and a sulfonic acid group, still further preferably one or more kind selected from the group consisting of a linear alkylbenzenesulfonate and a high molecular weight dispersant having a carboxy group, still more further preferably one or more kind selected from the group consisting of a linear alkylbenzenesulfonate and a high molecular weight dispersant having a carboxy group containing a structural unit derived from one or more kind of a carboxy group-containing vinyl monomer selected from the group consisting of acrylic acid, methacrylic acid, maleic aid, and maleic anhydride, and even further preferably one or more kind selected from the group consisting of sodium dodecylbenzenesulfonate, an acrylic acid homopolymer, a methacrylic acid homopolymer, an acrylic acid-maleic acid copolymer, a methacrylic acid-maleic acid copolymer, an acrylic acid-maleic anhydride copolymer, a methacrylic acid-maleic anhydride copolymer, and salts of the homopolymers and copolymers, from the standpoint of enhancing the dispersion stability of the microcapsules, and enhancing the long-term retainability.

The aqueous microcapsule dispersion liquid of the present invention may contain any other component than the components (A) to (D) depending on necessity. Examples of the other component include a colorant, an antiseptic, an antioxidant, an ultraviolet ray absorbent, a shell surface modifier, a thickener, a deposition aid, and a rheology modifier.

The aqueous microcapsule dispersion liquid of the present invention may be allowed to contain in advance a fabric softener, a fabric freshener, a fabric toughening agent, an enzyme, a builder liquid, a hair conditioner, a skin conditioner, a fragrance material, clay, zeolite, silicone, and the like, other than the components (A) to (D), depending on necessity, for blending the aqueous dispersion liquid in various formulations.

### [Method of producing Aqueous Microcapsule Dispersion Liquid]

The method of producing the aqueous microcapsule dispersion liquid of the present invention is not particularly limited. For example, the aqueous microcapsule dispersion liquid can be produced by a method including a step of mixing the component (A) which have been produced by known methods in advance, the component (B), and depending on necessity the component (C) or the component (D), and the aforementioned other component.

In particular, the method of producing the aqueous microcapsule dispersion liquid of the present invention is preferably a method including a step of adding the component (B) to an aqueous microcapsule dispersion containing the component (A), from the standpoint of enhancing the long-term retainability and the standpoint of the easiness of production.

The method of producing the aqueous microcapsule dispersion liquid of the present invention may include further adding the component (C) or the component (D), and the aforementioned other component depending on necessity in adding the component (B) to the aqueous microcapsule dispersion containing the component (A).

For example, in the case where the component (A) is silica capsules, the method of producing the aqueous microcapsule dispersion liquid of the present invention is preferably a method including a step of mixing an aqueous dispersion containing silica capsules as the component (A) and the component (B), and depending on necessity the component (C) or the component (D), and the aforementioned other component. The aqueous dispersion containing silica capsules can be obtained by the method described above.

While the order of addition of the components is not particularly limited, it is preferred that the component (B) and depending on necessity the component (C) are added in this order to the aqueous microcapsule dispersion containing the component (A) to regulate pH, and then the aforementioned other component is added thereto depending on necessity. The component (B) and the component (C) used depending on necessity each may be used in the form of an aqueous solution depending on necessity.

In the case where the aqueous microcapsule dispersion liquid contains the component (D), it is preferred that the component (D) is added to the aqueous microcapsule dispersion containing the component (A), then the component (B) and depending on necessity the component (C) are added in this order thereto to regulate pH, and then the aforementioned other component is added thereto depending on necessity. The component (D) may be used in the form of an aqueous solution depending on necessity.

The mixing temperature of the aqueous microcapsule dispersion containing the component (A) and the component (B), and the component (C) or the component (D), and the other component used depending on necessity is preferably 15°C or more, and more preferably 20°C or more, and is also preferably 35°C or less, and more preferably 30°C or less, from the standpoint of enhancing the dispersion stability of the microcapsules, and enhancing the long-term retainability.

The components may be mixed by using a known agitation device or the like.

### (Composition of Aqueous Microcapsule Dispersion Liquid)

The content or the blending amount of the component (A) in the aqueous microcapsule dispersion liquid according to the present invention is preferably 50% by mass or less, more preferably 45% by mass or less, further preferably 40% by mass or less, still further preferably 35% by mass or less, and still more further preferably 30% by mass or less, from the standpoint of reducing the viscosity of the aqueous dispersion liquid, and enhancing the handleability, and is also preferably 5% by mass or more, more preferably 10% by mass or more, further preferably 15% by mass or more, still further preferably 18% by mass or more, and still more further preferably 20% by mass or more, from the standpoint of facilitating the preparation of a liquid composition or a product using the aqueous dispersion liquid.

The content or the blending amount of the component (B) in the aqueous microcapsule dispersion liquid according to the present invention is preferably 0.1% by mass or more, more preferably 0.15% by mass or more, and further preferably 0.2% by mass or more, and is also preferably 1% by mass or less, more preferably 0.7% by mass or less, further preferably 0.5% by mass or less, still further preferably 0.4% by mass or less, and still more further preferably 0.3% by mass or less, from the standpoint of enhancing the long-term retainability.

The content or the blending amount of the component (B) per 100 parts by mass of the component (A) in the aqueous microcapsule dispersion liquid according to the present invention is preferably 0.1 part by mass or more, more preferably 0.5 part by mass or more, and further preferably 0.9 part by mass or more, from the standpoint of enhancing the long-term retainability, and is also preferably 5.0 parts by mass or less, more preferably 4.0 parts by mass or less, further preferably 3.0 parts by mass or less, and still further preferably 2.0 parts by mass or less, from the standpoint of suppressing the salt concentration in the aqueous dispersion liquid, and enhancing the dispersion stability of the microcapsules.

In the case where the aqueous microcapsule dispersion liquid according to the present invention contains the component (D), the content or the blending amount of the component (D) in the aqueous microcapsule dispersion liquid is preferably 0.05% by mass or more, more preferably 0.07% by mass or more, and further preferably 0.1% by mass or more, and is also preferably 3% by mass or less, more preferably 2% by mass or less, further preferably 1% by mass or less, still further preferably 0.7% by mass or less, and still more further preferably 0.5% by mass or less, from the standpoint of enhancing the dispersion stability of the microcapsules.

The content or the blending amount of the component (D) per 100 parts by mass of the component (A) in the aqueous microcapsule dispersion liquid according to the present invention is preferably 0.1 part by mass or more, more preferably 0.3 part by mass or more, and further preferably 0.5 part by mass or more, from the standpoint of enhancing the dispersion stability of the microcapsules, suppressing the occurrence of cleavage among the capsules due to the aggregation of the microcapsules, and enhancing the long-term retainability, and is also preferably 10 parts by mass or less, more preferably 9.0 parts by mass or less, further preferably 8.0 parts by mass or less, still further preferably 7.0 parts by mass or less, still more further preferably 5.0 parts by mass or less, even further preferably 3.0 parts by mass or less, even still further preferably 2.0 parts by mass or less, and even still more further preferably 1.7 parts by mass or less, from the standpoint of suppressing the foaming, and enhancing the handleability.

The pH of the aqueous microcapsule dispersion liquid according to the present invention is preferably 7.5 or more, more preferably 8.0 or more, further preferably 8.5 or more, and still further preferably 9.0 or more, and is also preferably 11.0 or less, more preferably 10.5 or less, and further preferably 10.0 or less, from the standpoint of regulating to a desired alkaline environment, and enhancing the long-term retainability, and the standpoint of the handleability. The pH of the aqueous microcapsule dispersion liquid can be measured by the method shown in the examples.

In the present invention, the component (C) is preferably contained or blended in the aqueous dispersion liquid to make the pH of the aqueous microcapsule dispersion liquid within the aforementioned range.

The viscosity at 25°C of the aqueous microcapsule dispersion liquid according to the present invention is preferably 1 mPa·s or more, more preferably 2 mPa·s or more, and further preferably 3 mPa s or more, and is also preferably 4,000 mPa·s or less, more preferably 2,000 mPa·s or less, further preferably 1,000 mPa·s or less, still further preferably 500 mPa·s or less, still more further preferably 100 mPa s or less, even further preferably 50 mPa s or less, and even still further preferably 30 mPa·s or less.

The viscosity at 25°C of the aqueous microcapsule dispersion liquid of the present invention can be measured by the method shown in the examples.

The aqueous microcapsule dispersion liquid according to the present invention is excellent in long-term retainability and can achieve both the good handleability and the low environmental burden simultaneously, and therefore can be used in various applications. Examples of the applications include perfumery and cosmetics, such as an emulsion lotion, a cosmetic liquid, a cosmetic lotion, a beauty lotion, a cosmetic cream, a gel formulation, a hair treatment, and a quasi drug; a fiber treatment agent, such as a detergent, a softener, and an anti-wrinkle spray; a sanitary product, such as a disposable diaper; and a perfuming agent, and the aqueous microcapsule dispersion liquid can be favorably applied to the production in these applications.

The aqueous microcapsule dispersion liquid according to the present invention is preferably used by being contained or by blending in a liquid composition, such as a detergent composition, a fiber treatment agent composition, a perfumery and cosmetic composition, a perfuming agent composition, and a deodorizer composition. The composition is preferably one or more kind selected from the group consisting of a detergent composition, such as a powder detergent composition and a liquid detergent composition; and a fiber treatment agent composition, such as a softener composition, more preferably a fiber treatment agent composition, and further preferably a softener composition.

### [Method of stabilizing Aqueous Microcapsule Dispersion Liquid]

The method of stabilizing an aqueous microcapsule dispersion liquid of the present invention is a method of stabilizing an aqueous microcapsule dispersion liquid containing a component (A) and a component (B) below from the standpoint of enhancing the long-term retainability:
component (A): microcapsules having a shell containing an inorganic material as a constitutional component, and a core containing one or more kind of an organic compound, inside the shell, and
component (B): one or more kind selected from the group consisting of an amino acid (B1), a polyhydroxyamine (B2), and a quaternary ammonium hydroxide (B3), and
the method includes adding the component (B) to an aqueous microcapsule dispersion containing the component (A), so as to stabilize the aqueous microcapsule dispersion liquid.

In the present invention, the term "stabilizing" means that the organic compound, such as a fragrance material, as an active ingredient encapsulated in the microcapsules contained in the aqueous microcapsule dispersion liquid is suppressed from being leaked, and thereby the organic compound encapsulated in the microcapsules is retained for a long period of time.

The method of stabilizing an aqueous microcapsule dispersion liquid of the present invention may include further adding the component (C) or the component (D), and the aforementioned other component depending on necessity in adding the component (B) to the aqueous microcapsule dispersion containing the component (A).

The descriptions of the components (A) to (D) and the other component herein are the same as the descriptions thereof for the aqueous microcapsule dispersion liquid, and therefore are omitted herein.

For example, in the case where the component (A) is silica capsules, the method of stabilizing an aqueous microcapsule dispersion liquid of the present invention is preferably a method including a step of mixing an aqueous dispersion containing silica capsules as the component (A) and the component (B), and depending on necessity the component (C) or the component (D), and the aforementioned other component. The aqueous dispersion containing silica capsules can be obtained by the method described above.

While the order of addition of the components is not particularly limited, it is preferred that the component (B) and depending on necessity the component (C) are added in this order to the aqueous microcapsule dispersion containing the component (A) to regulate pH, and then the aforementioned other component is added thereto depending on necessity. The component (B) and the component (C) used depending on necessity each may be used in the form of an aqueous solution depending on necessity.

In the case where the aqueous microcapsule dispersion liquid contains the component (D), it is preferred that the component (D) is added to the aqueous microcapsule dispersion containing the component (A), then the component (B) and depending on necessity the component (C) are added in this order thereto to regulate pH, and then the aforementioned other component is added thereto depending on necessity. The component (D) may be used in the form of an aqueous solution depending on necessity.

The mixing temperature of the aqueous microcapsule dispersion containing the component (A) and the component (B), and the component (C), the component (D), and the other component used depending on necessity is preferably 15°C or more, and more preferably 20°C or more, and is also preferably 35°C or less, and more preferably 30°C or less, from the standpoint of enhancing the dispersion stability of the microcapsules, and enhancing the long-term retainability.

The components may be mixed by using a known agitation device or the like.

In relation to the embodiments described above, the present invention further relates to the aqueous microcapsule dispersion liquids, the methods of producing an aqueous microcapsule dispersion liquid, and the method of stabilizing an aqueous microcapsule dispersion liquid shown below.
<1> An aqueous microcapsule dispersion liquid containing a component (A) and a component (B) below:
   component (A): microcapsules having a shell containing an inorganic material as a constitutional component, and a core containing one or more kind of an organic compound, inside the shell, and
   component (B): one or more kind selected from the group consisting of an amino acid (B1), a polyhydroxyamine (B2), and a quaternary ammonium hydroxide (B3).
<2> The aqueous microcapsule dispersion liquid according to the item <1>, wherein the component (B) has an acid dissociation exponent pKa at 25°C of at least one dissociation stages of preferably 7.5 or more, more preferably 8.0 or more, and further preferably 9.0 or more, and of preferably 13.0 or less, more preferably 12.0 or less, further preferably 11.0 or less, and still further preferably 10.0 or less.
<3> The aqueous microcapsule dispersion liquid according to the item <1> or <2>, wherein the component (B) is preferably one or more kind selected from the group consisting of the amino acid (B1) and the polyhydroxyamine (B2), more preferably contains the amino acid (B 1), and further preferably is the amino acid (B1).
<4> The aqueous microcapsule dispersion liquid according to any one of the items <1> to <3>, wherein the amino acid (B1) is preferably one or more kind selected from the group consisting of a neutral amino acid and an acidic amino acid, more preferably a neutral amino acid, further preferably a monoaminomonoacarboxylic acid, still further preferably one or more kind selected from the group consisting of glycine and alanine, and still more further preferably glycine.
<5> The aqueous microcapsule dispersion liquid according to any one of the items <1> to <4>, wherein the aqueous microcapsule dispersion liquid further contains an alkaline agent (provided that the component (B) is excluded) as a component (C).
<6> The aqueous microcapsule dispersion liquid according to the item <5>, wherein the component (C) is preferably one or more kind selected from the group consisting of a hydroxide, a silicate, a carbonate, and a phosphate of an alkali metal, and an organic amine, more preferably one or more kind selected from the group consisting of a silicate of an alkali metal and a monohydroxyalkylamine having 1 or more and 10 or less carbon atoms in total, further preferably one or more kind selected from the group consisting of sodium silicate and a monohydroxyalkylamine having 2 or more and 6 or less carbon atoms in total, and still further preferably one or more kind selected from the group consisting of JIS No. 2 sodium silicate and monoethanolamine.
<7> The aqueous microcapsule dispersion liquid according to any one of the items <1> to <6>, wherein the inorganic material is preferably a metal oxide containing a metal element or a semimetal element, more preferably an inorganic polymer formed through sol-gel reaction using a metal alkoxide (M(OR)ₓ) as a shell precursor, further preferably an inorganic polymer formed through sol-gel reaction using a metal alkoxide of one or more kind selected from the group consisting of silicon, aluminum, and titanium as a shell precursor, and still further preferably silica formed through sol-gel reaction using an alkoxysilane as a shell precursor.
<8> The aqueous microcapsule dispersion liquid according to the item <7>, wherein the alkoxysilane is preferably a tetraalkoxysilane.
<9> The aqueous microcapsule dispersion liquid according to any one of the items <1> to <6>, wherein the component (A) is microcapsules having a shell containing silica as a constitutional component, and a core containing one or more kind of an organic compound, inside the shell.
<10> The aqueous microcapsule dispersion liquid according to the item <9>, wherein the shell is a multilayer shell including an inner shell containing silica, which is a hydrolytic polycondensation product of an alkoxysilane, as a constitutional component, and an outer shell containing silica, which is a hydrolytic polycondensation product of an alkoxysilane, as a constitutional component, outside the inner shell.
<11> The aqueous microcapsule dispersion liquid according to any one of the items <1> to <10>, wherein the organic compound is preferably one or more kind selected from the group consisting of a fragrance material; a fragrance precursor; an oil; an antioxidant; an antibacterial agent; a fertilizer; a surface modifier for fibers, skin, hair, and the like; a cooling agent; a dye; a colorant; a silicone; a solvent; and an oil soluble polymer, more preferably one or more kind selected from the group consisting of a fragrance material, a fragrance precursor, an oil, an antioxidant, an antibacterial agent, a fertilizer, a surface modifier, and a solvent, further preferably one or more kind selected from the group consisting of a fragrance material, a fragrance precursor, an oil, an antioxidant, and a solvent, still further preferably one or more kind selected from the group consisting of a fragrance material, a fragrance precursor, and an oil, and still more further preferably one or more kind selected from a fragrance material and a fragrance precursor.
<12> The aqueous microcapsule dispersion liquid according to any one of the items <1> to <11>, wherein the organic compound has a cLogP value of preferably 1 or more, more preferably 2 or more, and further preferably 3 or more, and of preferably 30 or less, more preferably 20 or less, and further preferably 10 or less.
<13> The aqueous microcapsule dispersion liquid according to any one of the items <1> to <12>, wherein the microcapsules as the component (A) have a median diameter D₅₀ of preferably 100 µm or less, more preferably 75 µm or less, further preferably 50 µm or less, still further preferably 30 µm or less, and still more further preferably 10 µm or less, and of preferably 0.01 µm or more, more preferably 0.05 µm or more, further preferably 0.07 µm or more, still further preferably 0.1 µm or more, still more further preferably 0.5 µm or more, and even further preferably 1 µm or more.
<14> An aqueous microcapsule dispersion liquid containing a component (A,) a component (B), and a component (C) below:
   component (A): microcapsules having a shell containing silica as a constitutional component, and a core containing one or more kind of an organic compound, inside the shell,
   component (B): one or more kind selected from the group consisting of an amino acid (B1), a polyhydroxyamine (B2), and a quaternary ammonium hydroxide (B3),
   component (C): an alkaline agent (provided that the component (B) is excluded).
<15> The aqueous microcapsule dispersion liquid according to the item <14>, wherein the component (B) is preferably one or more kind selected from the group consisting of the amino acid (B1) and the polyhydroxyamine (B2), more preferably contains the amino acid (B 1), and further preferably is the amino acid (B1).
<16> The aqueous microcapsule dispersion liquid according to the item <14> or <15>, wherein the component (B1) is preferably one or more kind selected from the group consisting of a neutral amino acid and an acidic amino acid, more preferably a neutral amino acid, further preferably a monoaminomonoacarboxylic acid, still further preferably one or more kind selected from the group consisting of glycine and alanine, and still more further preferably glycine.
<17> The aqueous microcapsule dispersion liquid according to any one of the items <14> to <16>, wherein the component (C) is preferably one or more kind selected from the group consisting of a hydroxide, a silicate, a carbonate, and a phosphate of an alkali metal, and an organic amine, more preferably one or more kind selected from the group consisting of a silicate of an alkali metal and a monohydroxyalkylamine having 1 or more and 10 or less carbon atoms in total, further preferably one or more kind selected from the group consisting of sodium silicate and a monohydroxyalkylamine having 2 or more and 6 or less carbon atoms in total, and still further preferably one or more kind selected from the group consisting of JIS No. 2 sodium silicate and monoethanolamine.
<18> The aqueous microcapsule dispersion liquid according to any one of the items <1> to <17>, wherein a content or a blending amount of the component (A) in the aqueous microcapsule dispersion liquid is preferably 50% by mass or less, more preferably 45% by mass or less, further preferably 40% by mass or less, still further preferably 35% by mass or less, and still more further preferably 30% by mass or less, and is also preferably 5% by mass or more, more preferably 10% by mass or more, further preferably 15% by mass or more, still further preferably 18% by mass or more, and still more further preferably 20% by mass or more.
<19> The aqueous microcapsule dispersion liquid according to any one of the items <1> to <18>, wherein a content or a blending amount of the component (B) in the aqueous microcapsule dispersion liquid is preferably 0.1% by mass or more, more preferably 0.15% by mass or more, and further preferably 0.2% by mass or more, and is also preferably 1% by mass or less, more preferably 0.7% by mass or less, further preferably 0.5% by mass or less, still further preferably 0.4% by mass or less, and still more further preferably 0.3% by mass or less.
<20> The aqueous microcapsule dispersion liquid according to any one of the items <1> to <19>, wherein a content or a blending amount of the component (B) per 100 parts by mass of the component (A) in the aqueous microcapsule dispersion liquid is preferably 0.1 part by mass or more, more preferably 0.5 part by mass or more, and further preferably 0.9 part by mass or more, and is also preferably 5.0 parts by mass or less, more preferably 4.0 parts by mass or less, further preferably 3.0 parts by mass or less, and still further preferably 2.0 parts by mass or less.
<21> The aqueous microcapsule dispersion liquid according to any one of the items <1> to <20>, wherein the aqueous microcapsule dispersion liquid further contains a dispersant as a component (D).
<22> The aqueous microcapsule dispersion liquid according to the item <21>, wherein the component (D) is preferably an anionic surfactant, more preferably one or more kind selected from a low molecular weight dispersant having an anionic group and a high molecular weight dispersant having an anionic group, further preferably one or more kind selected from a linear alkylbenzenesulfonate and a high molecular weight dispersant having one or more kind of an anionic group selected from a carboxy group and a sulfonic acid group, still further preferably one or more kind selected from a linear alkylbenzenesulfonate and a high molecular weight dispersant having a carboxy group, still more further preferably one or more kind selected from a linear alkylbenzenesulfonate and a high molecular weight dispersant having a carboxy group containing a structural unit derived from one or more kind of a carboxy group-containing vinyl monomer selected from acrylic acid, methacrylic acid, maleic aid, and maleic anhydride, and even further preferably one or more kind selected from sodium dodecylbenzenesulfonate, an acrylic acid homopolymer, a methacrylic acid homopolymer, an acrylic acid-maleic acid copolymer, a methacrylic acid-maleic acid copolymer, an acrylic acid-maleic anhydride copolymer, a methacrylic acid-maleic anhydride copolymer, and salts of the homopolymers and copolymers.
<23> The aqueous microcapsule dispersion liquid according to the item <21> or <22>, wherein a content or a blending amount of the component (D) in the aqueous microcapsule dispersion liquid is preferably 0.05% by mass or more, more preferably 0.07% by mass or more, and further preferably 0.1% by mass or more, and is also preferably 3% by mass or less, more preferably 2% by mass or less, further preferably 1% by mass or less, still further preferably 0.7% by mass or less, and still more further preferably 0.5% by mass or less.
<24> The aqueous microcapsule dispersion liquid according to any one of the items <21> to <23>, wherein a content or a blending amount of the component (D) per 100 parts by mass of the component (A) in the aqueous microcapsule dispersion liquid is preferably 0.1 part by mass or more, more preferably 0.3 part by mass or more, and further preferably 0.5 part by mass or more, and is also preferably 10 parts by mass or less, more preferably 9.0 parts by mass or less, further preferably 8.0 parts by mass or less, still further preferably 7.0 parts by mass or less, still more further preferably 5.0 parts by mass or less, even further preferably 3.0 parts by mass or less, even still further preferably 2.0 parts by mass or less, and even still more further preferably 1.7 parts by mass or less.
<25> The aqueous microcapsule dispersion liquid according to any one of the items <1> to <24>, wherein the aqueous microcapsule dispersion liquid has a pH of preferably 7.5 or more, more preferably 8.0 or more, further preferably 8.5 or more, and still further preferably 9.0 or more, and of preferably 11.0 or less, more preferably 10.5 or less, and further preferably 10.0 or less.
<26> A method of producing the aqueous microcapsule dispersion liquid according to any one of the items <1> to <25>, including a step of mixing an aqueous dispersion containing the component (A) and the component (B).
<27> A method of producing the aqueous microcapsule dispersion liquid according to any one of the items <1> to <26>, including a step of mixing an aqueous dispersion containing the component (A), the component (B), and the component (C).
<28> A method of producing an aqueous microcapsule dispersion liquid containing a component (A) and a component (B) below:
   component (A): microcapsules having a shell containing an inorganic material as a constitutional component, and a core containing one or more kind of an organic compound, inside the shell, and
   component (B): one or more kind selected from the group consisting of an amino acid (B1), a polyhydroxyamine (B2), and a quaternary ammonium hydroxide (B3),
   the method of producing an aqueous microcapsule dispersion liquid including a step of adding the component (B) to an aqueous microcapsule dispersion containing the component (A).
<29> The method of producing an aqueous microcapsule dispersion liquid according to the item <28>, wherein the step is a step of adding the component (B) and the component (C) in this order to an aqueous microcapsule dispersion containing the component (A) to regulate pH.
<30> The method of producing an aqueous microcapsule dispersion liquid according to the item <28> or <29>, wherein the step is a step of adding the component (D) to an aqueous microcapsule dispersion containing the component (A), and then adding the component (B) and the component (C) in this order thereto to regulate pH.
<31> A method of stabilizing an aqueous microcapsule dispersion liquid containing a component (A) and a component (B) below:
   component (A): microcapsules having a shell containing an inorganic material as a constitutional component, and a core containing one or more kind of an organic compound, inside the shell, and
   component (B): one or more kind selected from the group consisting of an amino acid (B1), a polyhydroxyamine (B2), and a quaternary ammonium hydroxide (B3),
   the method of stabilizing an aqueous microcapsule dispersion liquid including adding the component (B) to an aqueous microcapsule dispersion containing the component (A), so as to stabilize the aqueous microcapsule dispersion liquid.
<32> The method of stabilizing an aqueous microcapsule dispersion liquid according to the item <31>, wherein the step is a step of adding the component (B) and the component (C) in this order to an aqueous microcapsule dispersion containing the component (A) to regulate pH.
<33> The method of stabilizing an aqueous microcapsule dispersion liquid according to the item <31> or <32>, wherein the step is a step of adding the component (D) to an aqueous microcapsule dispersion containing the component (A), and then adding the component (B) and the component (C) in this order thereto to regulate pH.

### Examples

The measurements in Examples and Comparative Examples were performed by the following methods.

### [Measurement of Median Diameter D₅₀]

The median diameter D₅₀ of the emulsion droplets and the median diameter D₅₀ of the microcapsules were measured by using a laser diffraction/scattering particle diameter distribution measuring device, "LA-960" (trade name, available from Horiba, Ltd.). In the measurement, a flow cell was used, water was used as a medium, and the refractive index of the dispersoid was set to 1.45-0i. The emulsion liquid or the aqueous dispersion containing the microcapsules was added to the flow cell, and measured at a concentration showing a transmittance of approximately 90%, so as to obtain the volume based median diameter D₅₀.

### [Measurement of pH]

The pH value at 25°C was measured by using a portable pH meter "D-71" (trade name, available from Horiba, Ltd.) using a pH electrode "9680S-10D" (trade name, available from Horiba, Ltd.).

### [Measurement of Viscosity]

The viscosity of the aqueous microcapsule dispersion liquid was measured by using an E-type viscometer (Model TVE35L, available from Toki Sangyo Co., Ltd.) using a cone rotor of 1°34' × R24 at a rotation speed of 20 rpm and a measurement temperature of 25°C.

### <Model Fragrance Material>

As the organic compound encapsulated in the microcapsules, a model fragrance material A (volume average cLogP: 3.9, specific gravity: 0.96) having the composition shown in Table 1 was used. The volume average cLogP value of the model fragrance material was calculated in such a manner that the cLogP values of the fragrance components contained in the model fragrance material were multiplied by the volume proportions thereof in the model fragrance material respectively, and the resulting values were summated. In this calculation, all the fragrance components that each had a content of 0.5% by mass or more in the model fragrance material A were considered, and even the fragrance component that had a content of less than 0.5% by mass in the model fragrance material A was included in the calculation in the case where the specific gravity and the cLogP value thereof were known.

**Table 1: Model Fragrance Material A**

| Name of fragrance component | Content (% by mass) | cLogP |
|---|---|---|
| Methyl dihydrojasmonate | 19.7 | 3.0 |
| γ-Decalactone | 13.0 | 2.6 |
| Ethylene brassylate | 11.5 | 4.7 |
| o-t-Butylcyclohexyl acetate | 10.6 | 4.4 |
| Amber core | 7.7 | 4.1 |
| Others | 37.5 | |

### (Synthesis of Component (A))

### Synthesis Example 1-1

### (Step 1)

0.91 g of Quartamin 60W (trade name, cetyltrimethylammonium chloride, active ingredient: 30% by mass, available from Kao Corporation) was diluted with 224.13 g of ion exchanged water to provide a water phase component. An oil phase component prepared by mixing 60.03 g of the model fragrance material A and 15.10 g of tetraethoxysilane (which may be hereinafter referred to as "TEOS") was added to the water phase component, and the mixed liquid was emulsified at room temperature (approximately 25°C) for 10 minutes with a homomixer (Model HM-310, available from HsiangTai Machinery Industry Co., Ltd.) set to a rotation number of 9,000 rpm and 10 minutes, so as to provide a emulsion liquid. The median diameter D₅₀ of the emulsion liquid at this time was 1.3 µm.

Subsequently, the resulting emulsion liquid was regulated for the pH thereof to 3.7 with a 1% by mass sulfuric acid aqueous solution, then transferred to a separable flask equipped with an agitation blade and a condenser, and agitated at 200 rpm for 24 hours while retaining the liquid temperature to 30°C, so as to provide an aqueous dispersion containing microcapsules having a core formed of the model fragrance material A and a first shell.

### (Step 2)

Subsequently, 8.4 g of TEOS was added to 280.0 g of the aqueous dispersion obtained in the step 1 in a dropwise manner over 420 minutes. After the dropwise addition, the mixture was further agitated for 17 hours to form a second shell encapsulating the first shell, resulting in an aqueous dispersion containing silica capsules (A-1) having the model fragrance material A encapsulated by amorphous silica in 21.7% by mass. The median diameter D₅₀ of the silica capsules (A-1) was 2.1 µm. The content of the silica capsules (A-1) in the aqueous dispersion described above is a calculated value from the blending formulation in synthesizing the silica capsules.

### (Production of Aqueous Microcapsule Dispersion Liquid)

### Examples 1 and 2

The component (B) and the component (C) were added in this order to an aqueous dispersion containing the component (A) at 20 to 25°C to make the composition shown in Table 2 to regulate the pH to the value shown in Table 2, and then Proxel BDN (trade name, active ingredient: 1,2-benzisothiazolin-3-one, available from Lonza Japan, Ltd. (which is hereinafter referred to as "Proxel BDN")) as an antiseptic was added thereto to make a concentration thereof in the aqueous dispersion liquid of 600 ppm for Example 1 or 1,000 ppm for Example 2, resulting in an aqueous microcapsule dispersion liquid.

The viscosity of the aqueous microcapsule dispersion liquid obtained in Example 1 was 4.5 mPa·s.

### Examples 3 to 7

The component (D), the component (B), and the component (C) were added in this order to an aqueous dispersion containing the component (A) at 20 to 25°C to make the composition shown in Table 2 to regulate the pH to 9.5, and then Proxel BDN as an antiseptic was added thereto to make a concentration thereof in the aqueous dispersion liquid of 1,000 ppm, resulting in an aqueous microcapsule dispersion liquid.

The aqueous microcapsule dispersion liquids obtained in Examples 3 to 7 each were stored at 20°C and observed for the occurrence of gelation and aggregation of the silica capsules, and after 14 days, gelation and aggregation of the silica capsules were not confirmed, which showed good dispersion stability of the microcapsules.

### Comparative Example 1

The component (C) was added to an aqueous dispersion containing the component (A) at 20 to 25°C to make the composition shown in Table 2 to regulate the pH to 9.5, and then Proxel BDN as an antiseptic was added thereto to make a concentration thereof in the aqueous dispersion liquid of 1,000 ppm, resulting in an aqueous microcapsule dispersion liquid.

### Comparative Example 2

The component (D) and the component (C) were added to an aqueous dispersion containing the component (A) at 20 to 25°C to make the composition shown in Table 2 to regulate the pH to 9.5, and then Proxel BDN as an antiseptic was added thereto to make a concentration thereof in the aqueous dispersion liquid of 1,000 ppm, resulting in an aqueous microcapsule dispersion liquid.

The details of the components shown in Table 2 are as follows.
Tris: trishydroxymethylaminomethane
No. 2 sodium silicate: 2Na₂O·5SiO₂ aqueous solution, active ingredient: 40% by mass (available from Fuji Chemical Co., Ltd.)
LAS-Na: sodium dodecylbenzenesulfonate (Neopelex G-25, trade name, active ingredient: 25% by mass, available from Kao Corporation)
Poiz 520: polycarboxylic acid type polymer surfactant (Poiz 520, trade name, active ingredient: 40% by mass, available from Kao Corporation)
Poiz 521: polycarboxylic acid type polymer surfactant (Poiz 521, trade name, active ingredient: 40% by mass, available from Kao Corporation)

### [Evaluation of Long-term Retainability of Fragrance Component]

The aqueous microcapsule dispersion liquids of Examples and Comparative Examples each were sealed in a screw tube and then allowed to stand still at 30°C. After standing still, the screw tube was stored for 14 days, and then 20 mg of the aqueous microcapsule dispersion liquid was collected and precisely weighed, diluted with 50 g of ion exchanged water, and then allowed to pass through a membrane filter (Omnipore, trade name, Model No. JAWP04700, available from Millipore Corporation), so as to recover the silica capsules on the membrane filter.

After further rinsing the silica capsules with 10 mL of ion exchanged water and then 10 mL of hexane on the membrane filter, the silica capsules were immersed in 10 mL of methanol containing dodecane as an internal standard in a concentration of 10 µg/mL, and then irradiated with an ultrasonic wave by using an ultrasonic wave irradiation device (Model 5510, available from Branson Ultrasonics Corporation) under conditions of an output power of 180 W and an oscillating frequency of 42 kHz for 60 minutes, so as to elute the fragrance material in the silica capsules. The solution was again allowed to pass through a membrane filter (Dismic, trade name, model No. 13JP020AN, available from Toyo Roshi Kaisha, Ltd.), and then the fragrance component contained in the solution was measured by gas chromatography to determine the amount α of the fragrance component encapsulated in the silica capsules.

Separately, 20 mg of the aqueous microcapsule dispersion liquid prepared in each of Examples and Comparative Examples before storing at 30°C was precisely weighed, immersed in 10 mL of methanol, and irradiated with an ultrasonic wave by using the ultrasonic wave irradiation device under conditions of an output power of 180 W and an oscillating frequency of 42 kHz for 60 minutes, so as to elute the fragrance material in the silica capsules. The solution was allowed to pass through a membrane filter (Dismic, trade name, model No. 13JP020AN, available from Toyo Roshi Kaisha, Ltd.), and then the fragrance component contained in the solution was measured by gas chromatography to determine the amount β of the fragrance component contained in the aqueous microcapsule dispersion liquid.

The retention rate of methyl dihydroj asmonate as the fragrance component contained in the model fragrance material A was calculated according to the following expression for evaluating the long-term retainability.

For convenience in analysis, a retention rate of methyl dihydrojasmonate exceeding 100% is assumed to be a retention rate of methyl dihydrojasmonate of 100%.

The results are shown in Table 2 below.

Retention rate of methyl dihydrojasmonate (%) = ((amount α of methyl dihydrojasmonate encapsulated in silica capsules after storing) / (amount β of methyl dihydrojasmonate contained in aqueous microcapsule dispersion liquid)) × 100

**Table 2**

| | | Aqueous microcapsule dis version liquid | | | | | | | | | Long-term retainability |
|---|---|---|---|---|---|---|---|---|---|---|---|
| | | Component (A) | | Component (B) | | | Component (C) | Component (D) | | pH | Retention rate of fragrance component (%) (storing temperature: 30°C, storing time: 14 days) |
| | | Kind | Content (blended amount) (% by mass) | Kind | Acid dissociation exponent at 25°C pKa | Content (blended amount) (% by mass) | Kind | Kind | Content (blended amount) (% by mass) *1 | | |
| Example | 1 | silica capsules (A-1) | 21.1 | glycine | 9.24 | 0.23 | monoethanolamine | - | - | 9.6 | 97 |
| | 2 | silica capsules (A-1) | 21.0 | alanine | 9.48 | 0.26 | monoethanolamine | - | - | 9.5 | 100 |
| | 3 | silica capsules (A-1) | 20.8 | glycine | 9.24 | 0.23 | monoethanolamine | LAS-Na | 0.30 | 9.5 | 100 |
| | 4 | silica capsules (A-1) | 20.9 | glycine | 9.24 | 0.22 | No. 2 sodium silicate | Poiz 520 | 0.05 | 9.5 | 88 |
| | | | | | | | | Poiz 521 | 0.11 | | |
| | 5 | silica capsules (A-1) | 21.0 | glycine | 9.24 | 0.22 | monoethanolamine | Poiz 520 | 0.05 | 9.5 | 94 |
| | | | | | | | | Poiz 521 | 0.10 | | |
| | 6 | silica capsules (A-1) | 20.9 | Tris | 8.95 | 0.36 | monoethanolamine | Poiz 520 | 0.05 | 9.5 | 90 |
| | | | | | | | | Poiz 521 | 0.10 | | |
| | 7 | silica capsules (A-1) | 20.7 | Tris | 8.95 | 0.35 | monoethanolamine | LAS-Na | 0.31 | 9.5 | 85 |
| Comparative Example | 1 | silica capsules (A-1) | 21.6 | - | - | | monoethanolamine | - | - | 9.5 | 82 |
| | 2 | silica capsules (A-1) | 21.3 | - | - | | monoethanolamine | LAS-Na | 0.30 | 9.5 | 56 |

| | | | | | | | | | | | |
|---|---|---|---|---|---|---|---|---|---|---|---|
| * 1: Content (blended amount) of active ingredient (% by mass) | | | | | | | | | | | |

It is understood from Table 2 that the aqueous microcapsule dispersion liquids of Examples are excellent in long-term retainability of the encapsulated fragrance component, as compared to those of Comparative Examples.

It is also understood that the aqueous microcapsule dispersion liquids of Examples 3 and 7 are excellent in long-term retainability of the encapsulated fragrance component, as compared to Comparative Example 2 using the same component (D).

### Industrial Applicability

The present invention can provide an aqueous microcapsule dispersion liquid that is capable of retaining an encapsulated organic compound, such as a fragrance material, as an active ingredient for a long period of time. Accordingly, the present invention can provide an aqueous microcapsule dispersion liquid that is free from thickening due to gelation or aggregation and has good handleability in imparting various functions, for example, imparting fragrance, to various products, such as a laundry product, a personal care product, a cosmetic product, and a household liquid product, and the aqueous microcapsule dispersion liquid can be widely utilized.

## Claims

1. An aqueous microcapsule dispersion liquid comprising a component (A) and a component (B) below:
component (A): microcapsules having a shell comprising an inorganic material as a constitutional component, and a core comprising one or more kind of an organic compound, inside the shell, and
component (B): one or more kind selected from the group consisting of an amino acid (B1), a polyhydroxyamine (B2), and a quaternary ammonium hydroxide (B3).

2. The aqueous microcapsule dispersion liquid according to claim 1, wherein the component (B) has an acid dissociation exponent pKa at 25°C of at least one dissociation stages of 7.5 or more and 13.0 or less.

3. The aqueous microcapsule dispersion liquid according to claim 1 or 2, wherein the component (B) comprises the amino acid (B1).

4. The aqueous microcapsule dispersion liquid according to claim 3, wherein the amino acid (B1) is one or more kind selected from the group consisting of glycine and alanine.

5. The aqueous microcapsule dispersion liquid according to any one of claims 1 to 4, further comprising an alkaline agent (provided that the component (B) is excluded) as a component (C).

6. The aqueous microcapsule dispersion liquid according to claim 5, wherein the component (C) is one or more kind selected from the group consisting of a hydroxide, a silicate, a carbonate, and a phosphate of an alkali metal, and an organic amine.

7. The aqueous microcapsule dispersion liquid according to any one of claims 1 to 6, wherein the aqueous microcapsule dispersion liquid has a pH of 7.5 or more and 11.0 or less.

8. The aqueous microcapsule dispersion liquid according to any one of claims 1 to 7, wherein a content of the component (B) in the aqueous microcapsule dispersion liquid is 0.1% by mass or more and 1% by mass or less.

9. The aqueous microcapsule dispersion liquid according to any one of claims 1 to 8, wherein a content of the component (A) in the aqueous microcapsule dispersion liquid is 5% by mass or more and 50% by mass or less.

10. The aqueous microcapsule dispersion liquid according to any one of claims 1 to 9, further comprising a dispersant as a component (D).

11. The aqueous microcapsule dispersion liquid according to claim 10, wherein a content of the component (D) in the aqueous microcapsule dispersion liquid is 0.05% by mass or more and 3% by mass or less.

12. The aqueous microcapsule dispersion liquid according to any one of claims 1 to 11, wherein the component (A) is microcapsules having a shell comprising silica as a constitutional component, and a core comprising one or more kind of an organic compound, inside the shell.

13. The aqueous microcapsule dispersion liquid according to any one of claims 1 to 12, wherein the microcapsules as the component (A) have a median diameter D₅₀ of 0.1 µm or more and 50 µm or less.

14. The aqueous microcapsule dispersion liquid according to any one of claims 1 to 13, wherein the organic compound is one or more kind selected from the group consisting of a fragrance material, a fragrance precursor, an oil, an antioxidant, and a solvent.

15. A method of producing an aqueous microcapsule dispersion liquid comprising a component (A) and a component (B) below:
component (A): microcapsules having a shell comprising an inorganic material as a constitutional component, and a core comprising one or more kind of an organic compound, inside the shell, and
component (B): one or more kind selected from the group consisting of an amino acid (B1), a polyhydroxyamine (B2), and a quaternary ammonium hydroxide (B3),
the method of producing an aqueous microcapsule dispersion liquid comprising a step of adding the component (B) to an aqueous microcapsule dispersion comprising the component (A).

16. A method of stabilizing an aqueous microcapsule dispersion liquid comprising a component (A) and a component (B) below:
component (A): microcapsules having a shell comprising an inorganic material as a constitutional component, and a core comprising one or more kind of an organic compound, inside the shell, and
component (B): one or more kind selected from the group consisting of an amino acid (B1), a polyhydroxyamine (B2), and a quaternary ammonium hydroxide (B3),
the method of stabilizing an aqueous microcapsule dispersion liquid comprising adding the component (B) to an aqueous microcapsule dispersion comprising the component (A), so as to stabilize the aqueous microcapsule dispersion liquid.
